# EUROPEAN PATENT APPLICATION

(11) **EP 2 662 092 A1**
(43) Date of publication of application: **13.11.2013**
(21) Application number: 12732271.7
(22) Date of filing: 05.01.2012
(51) Int. Cl.: A61K 45/00, A61K 31/235, A61K 31/7028, A61K 39/395, A61P 31/04, G01N 33/68

(54) **METHOD FOR SELECTING PATIENT TO BE GIVEN DRUG FOR TREATING SEPTICEMIA**

(30) Priority: 06.01.2011 JP 2011000943
(71) Applicant: Mochida Pharmaceutical Co., Ltd., Tokyo 160-8515 (JP)
(72) Inventor: IMAI Mariko, Tokyo 160-8515 (JP); SHIRAKAWA Kamon, Tokyo 160-8515 (JP)
(74) Representative: Grünecker, Kinkeldey, Stockmair & Schwanhäusser
(86) International application number: PCT/JP2012/050057
(87) International publication number: WO 2012/093681

(57) **Abstract**

The present invention is a method for selecting a patient to be given a drug for treating septicemia the mode of action of which is to interrupt toll-like receptor 4-derived intracellular signaling. The provided method comprises a step for measuring sCD14-ST in a blood sample from a subject, a step for comparing the measured value with a cut-off value, and a step for selecting a patient as the patient to be given the drug for the treatment of septicemia when the measured value of the sample of that patient is positive in comparison to the cut-off value. The present invention makes it possible to provide a drug to be given to a specific patient to treat septicemia, the mode of action of which is to interrupt toll-like receptor 4-derived intracellular signaling, and to appropriately select the patient to be given the drug and the administration timing or dose.

## Description

The present invention relates to a therapeutic agent for sepsis administered to a specific patient and having an action mechanism that inhibits occurrence of intracellular signaling derived from Toll-like receptor 4, a method for selecting a patient to be administered the therapeutic agent, a method for determining a timing for administering the therapeutic agent for sepsis, and a method for setting a dosage of the therapeutic agent for sepsis. The present invention also relates to a sepsis treating method applied to a selected patient.

### BACKGROUND ART

Sepsis is defined as a disease that has an infectious cause and shows the pathology of systemic inflammatory response syndrome (SIRS) (see Non-Patent Document 1). Initial symptoms found include ague, sweating, fever, and decrease in the blood pressure, and when various inflammatory mediators and blood coagulation factors increase in the whole body, disturbance in the microcirculation occurs, and this results in deterioration of the pathology, causing tissue and organ failures, which often lead to continuous onset of multiple organ failure or septic shock, leading to death.

The onset of sepsis is triggered by the action of the constituents of the infectious bacteria such as lipopolysaccharide (LPS) of Gram-negative bacteria and lipoteichoic acid (LTA) of Gram-positive bacteria on leukocytes (monocytes/macrophages and neutrophils) or vascular endothelial cells, which in turn causes production of various inflammatory mediators. Recent studies revealed that Toll-like receptor 4 (TLR4) plays an important role in activation of such target cells by constituents of bacteria. Specifically, LPS binds to an LPS-binding protein (LBP), and the LPS-LBP complex binds to CD14 on the cell membrane. The LPS-LBP-CD14 complex binds to TLR4, but TLR4 needs intermediacy of MD-2 in order to recognize the complex. TLR4 dimerizes upon recognizing LPS, and an intracellular signal is generated, leading to production of various kinds of cytokine (Non-Patent Document 2).

As the role played by TLR4 in the pathology of sepsis became clear, research and development of therapeutic agents for sepsis targeting TLR4 advanced (Non-Patent Document 3). E5564 (eritoran tetrasodium) is an analog of lipid A, a constituent of LPS. E5564 binds to a hydrophobic pocket of MD-2 and functions as an LPS antagonist to inhibit dimerization of TLR4 and occurrence of an intracellular signal. In a clinical test of E5564, E5564 was administered to severe septic patients whose mortality risk was estimated at 20 % to 80 % by APACHE II Score, with the result that while a tendency to reduce the mortality rate was observed in the high-dosage group, it was of no statistic significance (Non-Patent Document 4). Resatorvid (TAK-242) binds to Cys747 in the TLR4 intracellular domain to inhibit the binding of an intracellular adapter molecule and TLR4 and the occurrence of an intracellular signal. In a clinical test of Resatorvid, Resatorvid was administered to severe septic patients who exhibited a symptom of septic shock or respiratory failure, with the result that Resatorvid failed to suppress the serum IL-6 level, and that while a tendency to reduce the mortality rate was observed among the high-dosage group, it was of no statistic significance (Non-Patent Document 5). About the above clinical test using Resatorvid, an observation has been made that the effects of the drug may have been reduced by an action mechanism in which TLR4 is a factor involved in the early stages of sepsis and by an inappropriate selection of patients failing to meet the patient selection standards according to which patients should be administered Resatorvid within 36 hours of confirmation of such severe symptoms as septic shock and respiratory failure (Non-Patent Document 6).

Early-stage detection of sepsis and classification of the severity are of critical importance in order to enhance the possibility of providing a timely and sepsis-specific treatment. sCD14-ST (soluble CD14 antigen subtype) is a sepsis-specific marker whose blood concentration specifically increases in a septic patient and is known to be useful for early-stage detection of sepsis and evaluation of severity (Patent Document 1, Patent Document 2, and Patent Document 3). There are presently known about 170 kinds of sepsis-related biomarkers, among which procalcitonin (PCT) and C-reactive protein (CRP) are most widely used but considered to be insufficient for sepsis-specific diagnosis and prognostic prediction. Currently, distinguishing sepsis at its early stages from other non-infectious systemic inflammatory response is difficult, and a better sepsis marker is sought (Non-Patent Document 7).

In order to enhance the efficacy of a therapeutic agent for sepsis having an action mechanism that inhibits occurrence of TLR4-derived intracellular signaling, such agent needs to be administered to a patient at a timing that suits the action mechanism of the therapeutic agent for sepsis. However, there has been known no specific index or biomarker useful for determining a patient to be administered a therapeutic agent for sepsis and administration timing.

### CITATION LIST

### Patent Document

Patent Document 1: WO2004/044005
Patent Document 1: WO2005/108429
Patent Document 1: JP 2005-106694 A

### Non-Patent Document

[Non-Patent Document 1] The ACCP/SCCM Consensus Conference Committee. Chest, 101,pp1644-1655 (1992)
[Non-Patent Document 2]Kensuke Miyake, International immunopharmacology, 3, pp119-128 (2003)
[Non-Patent Document 3] X. Wittebole, et al., Mediators of Inflammation, Volume 2010, Article ID 568396 (2010)
[Non-Patent Document 4] Mark Tidswell, et al., Crit Care Med, 38, pp72-83 (2010)
[Non-Patent Document 5] Todd W. Rice, et al., Crit Care Med, 38, pp1685-1694 (2010)
[Non-Patent Document 6] Jorge I. F. Salluh, et al., Crit Care Med, 38, pp1749-1750 (2010)
[Non-Patent Document 7] Charalampos Pierrakos, et al., Critical Care, 14, R15 (2010)

### SUMMARY OF INVENTION

### TECHNICAL PROBLEMS

The present invention provides a method for selecting a patient to be administered a therapeutic agent for sepsis having an action mechanism that inhibits occurrence of TLR4-derived intracellular signaling, a method for determining a timing for administering the therapeutic agent for sepsis, and a method for setting a dosage of the therapeutic agent for sepsis. The present invention also provides a sepsis treating method applied to a selected patient.

### SOLUTION TO PROBLEMS

The inventors of the present invention found that, in order to enhance the efficacy of a therapeutic agent for sepsis having an action mechanism that inhibits occurrence of TLR4-derived intracellular signaling, it is useful to measure sCD14-ST in the blood of a subject, select a patient to be administered the therapeutic agent for sepsis using the sCD14-ST concentration in the blood of the patient as index, determine an administration timing, and set a dosage. The present invention has been thus completed.
The present invention is described in further detail below.

(1) A therapeutic agent for sepsis having an action mechanism that inhibits occurrence of Toll-like receptor 4-derived intracellular signaling, the therapeutic agent being administered to a subject suspected to have sepsis when a measured value obtained by measuring sCD14-ST in a blood specimen derived from the subject and compared with a cut-off value tests positive in comparison with the cut-off value (herein, the measured value is positive when it is equal to or higher than the cut-off value; the same applies below).
(2) A therapeutic agent for sepsis having an action mechanism that inhibits occurrence of Toll-like receptor 4-derived intracellular signaling, a dosage of the therapeutic agent being set according to a concentration of sCD14-ST in a blood specimen derived from a subject suspected to have sepsis when a measured value of the specimen obtained by measuring sCD14-ST in the blood specimen and compared with a cut-off value tests positive in comparison with the cut-off value.
(3) The therapeutic agent for sepsis having the action mechanism that inhibits occurrence of Toll-like receptor 4-derived intracellular signaling described in (1) or (2) above, wherein the therapeutic agent for sepsis having the action mechanism that inhibits occurrence of Toll-like receptor 4-derived intracellular signaling is at least one selected from the group consisting of Resatorvid, E5564, and F1024S-D2(3).

(4) A method for selecting a patient to be administered a therapeutic agent for sepsis having an action mechanism that inhibits occurrence of Toll-like receptor 4-derived intracellular signaling, the method comprising a step of measuring sCD14-ST in blood derived from a subject to obtain a measured value, a step of comparing the measured value with a cut-off value, and a step of selecting the patient to be administered the therapeutic agent for sepsis when the measured value of the specimen tests positive in comparison with the cut-off value.
(5) A method for determining a timing for administering a therapeutic agent for sepsis having an action mechanism that inhibits occurrence of Toll-like receptor 4-derived intracellular signaling, the method comprising a step of measuring sCD14-ST in blood derived from a subject to obtain a measured value, a step of comparing the measured value with a cut-off value, and a step of selecting the patient to be administered the therapeutic agent for sepsis when the measured value of the specimen tests positive in comparison with the cut-off value.
(6) A method for setting a dosage of a therapeutic agent for sepsis having an action mechanism that inhibits occurrence of Toll-like receptor 4-derived intracellular signaling, the method comprising a step of measuring sCD14-ST in blood derived from a subject to obtain a measured value, a step of comparing the measured value with a cut-off value, a step of selecting a patient to be administered the therapeutic agent for sepsis when the measured value of the specimen tests positive in comparison with the cut-off value, and a step of setting a dosage of the therapeutic agent for sepsis based on the sCD14-ST concentration in the specimen.

(7) The method described in any one of (4) to (6) above, wherein the therapeutic agent for sepsis having the action mechanism that inhibits occurrence of Toll-like receptor 4-derived intracellular signaling is at least one selected from the group consisting of Resatorvid, E5564, and F1024S-D2(3).
(8) The method described in any one of (4) to (6) above, wherein the therapeutic agent for sepsis having the action mechanism that inhibits occurrence of Toll-like receptor 4-derived intracellular signaling is Resatorvid.
(9) The method described in any one of (4) to (6) above, wherein the therapeutic agent for sepsis having the action mechanism that inhibits occurrence of Toll-like receptor 4-derived intracellular signaling is E5564.
(10) The method described in any one of (4) to (6) above, wherein the therapeutic agent for sepsis having the action mechanism that inhibits occurrence of Toll-like receptor 4-derived intracellular signaling is F1024S-D2(3).

The present invention also provides a sepsis treating method applied to a patient selected by the above method.
(11) A sepsis treating method comprising administering a therapeutic agent for sepsis having an action mechanism that inhibits occurrence of Toll-like receptor 4-derived intracellular signaling to a patient selected by the method described in (1) above.
(12) A sepsis treating method comprising administering a therapeutic agent for sepsis having an action mechanism that inhibits occurrence of Toll-like receptor 4-derived intracellular signaling to a patient selected by the method described in (1) above at a timing determined by the method described in (2) above.
(13) A sepsis treating method comprising administering a therapeutic agent for sepsis having an action mechanism that inhibits occurrence of Toll-like receptor 4-derived intracellular signaling to a patient selected by the method described in (1) above in a dosage set by the method described in (3) above.
(14) The method described in any one of (11) to (13) above, wherein the therapeutic agent for sepsis having the action mechanism that inhibits occurrence of Toll-like receptor 4-derived intracellular signaling is at least one selected from the group consisting of Resatorvid, E5564, and F1024S-D2(3).

### ADVANTAGEOUS EFFECTS OF INVENTION

According to the present invention, use of the sCD14-ST concentration in the blood of a patient as index permits early selection of a patient to be administered a therapeutic agent for sepsis having an action mechanism that inhibits occurrence of TLR4-derived intracellular signaling and enables administration of the therapeutic agent for sepsis to the patient at a timing that suits the action mechanism of the therapeutic agent for sepsis, thereby enhancing the efficacy of the therapeutic agent for sepsis. According to the invention, setting a dosage of the therapeutic agent for sepsis using the sCD14-ST concentration in the blood of a patient as index is also made possible, which enhances the therapeutic efficacy of the therapeutic agent for sepsis.

### BRIEF DESCRIPTION OF DRAWINGS

[FIG. 1] FIG. 1 shows survival rates in a Resatorvid-administered rabbit CLP model in Example 4. The horizontal axis indicates the time elapsed after surgery; the vertical axis indicates survival rate.
[FIG. 2] FIGS. 2A and 2B show changes in platelet count (FIG. 2A) and changes in leukocyte count (FIG. 2B) in the Resatorvid-administered rabbit CLP model in Example 4. The horizontal axis indicates the time elapsed after surgery; the vertical axis indicates leukocyte count.
[FIG. 3] FIG. 3 shows changes with time in the number of individuals of the Resatorvid-administered rabbit CLP model in Example 4 that tested positive with respect to the cut-off values of the sCD14-ST concentration and the body temperature elevation. The solid lines indicate changes observed where the sCD14-ST concentration was used as index; the broken lines indicate changes where elevation in body temperature was used as index. The horizontal axis indicates the time elapsed after surgery; the vertical axis indicates the ratio of the individuals that tested positive.

### DESCRIPTION OF EMBODIMENTS

Next, the present invention is described in detail.
The present invention provides a method for selecting a patient to be administered a therapeutic agent for sepsis having an action mechanism that inhibits occurrence of TLR4-derived intracellular signaling, a method for determining a timing for administering the therapeutic agent for sepsis, and a method for setting a dosage of the therapeutic agent for sepsis.

The therapeutic agent for sepsis having an action mechanism that inhibits occurrence of TLR4-derived intracellular signaling is a substance having an activity to inhibit at least one stage selected from the group consisting of a stage where LPS binds to LBP, a stage where the LPS-LBP complex binds to CD14 on the cell membrane, a stage where the LPS-LBP-CD14 complex binds to TLR4, a stage where the TLR4 recognizes the complex by the intermediacy of MD-2, a stage where the TLR4 dimerizes upon recognizing LPS, and a stage where an intracellular adapter molecule binds to the intracellular domain of the dimerized TLR4, and inhibits occurrence of TLR4-derived intracellular signaling. For simplicity, the therapeutic agent may be referred to as therapeutic agent for sepsis targeting TLR4 or TLR4-related therapeutic agent for sepsis. Representative examples of the adapter molecule herein include MyD88 (Myeloid Differentiation Protein-88), TIRAP/Mal (TIR domain-containing adaptor protein/MyD88-adaptor-like), TRIF (TIR domain-containing adaptor inducing IFN-β), and TRAM (TRIF-related adaptor molecule).

The activity to inhibit occurrence of TLR4-derived intracellular signaling may be confirmed using, for example, a cell that has expressed CD14 and TLR4 and hence admits LPS stimulation, by verifying whether production of a cytokine such as IL-6, IL-8, TNF-α, IL-1β, or IFN generated as a result of occurrence of TLR4-derived intracellular signaling or activation of a downstream signaling factor such as NF-κB is suppressed.

Examples of therapeutic agents for sepsis having an action mechanism that inhibits occurrence of TLR4-derived intracellular signaling include proteins such as soluble CD14, soluble MD-2, soluble TLR4, and modified forms thereof; anti-CD14 antibody, anti-MD-2 antibody, anti-TLR4 antibody, and modified forms thereof; an LPS antagonist, and a substance that binds to the intracellular domain of TLR4. Preferred examples thereof include F1024S-D2(3), which is a modified form of anti-CD14 antibody; E5564, which is an LPS antagonist; and Resatorvid, which is a substance that binds to the intracellular domain of TLR4.

F1024S-D2(3) is a modified form of an anti-CD14 antibody in which the domain 2 of urinary trypsin inhibitor (UTI) is bound to the anti-CD14 antibody. Details are described in the international publication WO2006/129849. Specifically, F1024S-D2(3) is a fusion protein containing a polypeptide having an amino acid sequence shown in FIG. 12 or FIG. 47 of the international publication WO2006/129849. F1024S-D2(3) is a therapeutic agent for sepsis that binds to CD14 to inhibit interaction with TLR4 and thereby inhibits occurrence of TLR4-derived intracellular signaling. F1024S-D2(3) has activities to suppress production of IL-6 induced by LPS stimulation to a human vascular endothelial cell, to improve the survival rate of an LPS-induced sepsis rabbit model, and to improve the survival rate of a cecal ligation and puncture-induced peritonitis rabbit model.

E5564 (α-D-Glucopyranose, 3-O-decyl-2-deoxy-6-O-[2-deoxy-3-O-[(3R)-3-methoxydecyl]-6-O-methyl-2-[[(11Z)-1-oxo-11-octadecenyl]amino]-4-O-phosphono-β-D-glucopyranosyl]-2-[(1,3-dioxotetradecyl)amino]-1-(dihydrogen phosphate), tetrasodium salt) is an analog of lipid A, a constituent of LPS, and is a therapeutic agent for sepsis that binds to a hydrophobic pocket of MD-2 and functions as LPS antagonist to inhibit dimerization of TLR4 and hence occurrence of TLR4-derived intracellular signaling.

Resatorvid (ethyl (6R)-6-[N-(2-chloro-4-fluorophenyl)sulfonyl]cyclohex-1-ene-1-carboxylate) is a therapeutic agent for sepsis that binds to Cys747 in the intracellular domain of TLR4 to inhibit the binding of intracellular adapter molecules and TLR4 and hence occurrence of TLR4-derived intracellular signaling.

According to the method of the invention, the sCD14-ST concentration in the blood of a patient is used as index in selecting a patient to be administered the therapeutic agent for sepsis having an action mechanism that inhibits occurrence of TLR4-derived intracellular signaling. sCD14-ST is a molecular species of a soluble CD14 protein that 1) is electrophoresed by SDS-PAGE to a molecular weight of 13 +/- 2 kDa under non-reducing conditions, 2) has on its N terminal sequence the amino acid sequence defined in SEQ ID NO. 1, and 3) specifically binds to an antibody prepared using the peptide comprising 16 amino acid residues defined in SEQ ID NO:2 as the antigen. Details are described in the international publication WO2005/108429.

The patient in the aspect of the present invention refers to a septic patient or a subject suspected to have sepsis. A subject suspected to have sepsis may be a subject suspected to have sepsis based on clinical findings. According to an example of clinical findings, a subject is suspected to have sepsis when the subject meets at least two items of the SIRS diagnostic criteria [1] to [4]. Verification of the infection by means of, for example, blood culture is not essential. sCD14-ST is produced in the phagocytosis process by a phagocyte, which is a biological defense mechanism acting on infection the earliest (see international publication WO2009/142303). Accordingly, measuring sCD14-ST in a subject suspected of infection enables verification of infection and, hence, of diagnosis of sepsis. Therefore, a subject suspected to have sepsis is a subject to whom the method of the invention may be suitably applied. The blood to be used may be any one of whole blood, plasma, and serum. The specimen herein refers to a blood specimen.

The method for selecting a patient to be administered the therapeutic agent for sepsis according to the invention comprises 1) measuring sCD14-ST in the blood derived from a subject, 2) comparing the measured value with a cut-off value, and 3) selecting the patient to be administered the therapeutic agent for sepsis when the measured value of the specimen is equal to or higher than the cut-off value and thus tests positive in comparison with the cut-off value.

In the step of measuring sCD14-ST in the blood derived from a subject, sCD14-ST in the blood may be measured by a known method. For example, the immunoassay for specifically detecting sCD14-ST described in the international publication WO2005/10842 may be used. Specifically, a preferred example thereof is a sandwich immunoassay using a combination of an antibody prepared by using the peptide comprising 16 amino acid residues defined in SEQ ID NO:2 in the Sequence Listing as the antigen and an antibody that binds to a peptide having an amino acid sequence from positions 17 to 26 defined in SEQ ID NO. 3 or an antibody that competes with the antibody (F1106-13-3 antibody or F1031-8-3 antibody).

2) In the step of comparing the measured value with the cut-off value, the measured value is compared with a predetermined cut-off value. Preferably, the cut-off value used in the present invention is so set as to suit the therapeutic agent for sepsis. For example, measured values of sCD14-ST in the blood of a plurality of normal persons are previously obtained, and the average, the median, or a range of the measured values thereof is calculated to obtain a normal person's standardized value or a normal person's range of value and use it as a normal person's standard value. The cut-off value may be, for example, the normal person's average + 2SD, twice the normal person's average, or twice (the normal person's average + 2SD). The SD herein means a standard deviation. An index such as a normal person's average + 3SD, or 2.5 times, 3 times, 3.5 times, or 4 times a normal person's average, for example, may be used so as to select a patient having a high degree of requirement and/or urgency for administration of the therapeutic agent for sepsis. The cut-off value may be so set as to increase the sensitivity and specificity of sepsis detection by comparing normal persons' previously measured sCD14-ST values with septic patients' sCD14-ST values. The cut-off value according to a quantitative method, whereby the cut-off value is predetermined numerically, may be, for example, 400 pg/mL, preferably 500 pg/mL. A cut-off value for selecting a patient with a high degree of urgency may be, for example, 1000 pg/mL, preferably 1800 pg/mL.

3) When the measured value of a specimen tests positive in comparison with the cut-off value, the subject is selected as a patient to be administered the therapeutic agent for sepsis in the step of selecting a patient to be administered the therapeutic agent for sepsis based on the result of comparison made in the step 2) above. That the measured value of a specimen tests positive in comparison with the cut-off value herein means that the measured value of a specimen is equal to or greater than the cut-off value. Where sCD14-ST is measured by a quantitative method, when the sCD14-ST concentration in a specimen indicates a value that is equal to or greater than the cut-off value that is set in the step 2), the subject from whom the specimen was taken is selected as a patient to be administered the therapeutic agent for sepsis. Where sCD14-ST is measured by a semiquantitative method, the sCD14-ST concentration is rated with such degrees as 0, 1, 2, 3 or -, +, ++, +++, and the like. The degrees correlate with the sCD14-ST concentrations detected by the quantitative method, so that whether a measured value is equal to or greater than the cut-off value may be judged based on the correlation between the rating by degree given according to the semiquantitative method and the sCD14-ST concentration determined by the quantitative method. Alternatively, a measured value that is less than the cut-off value may be allotted a rating such as 0 or -. Where sCD14-ST is measured by a qualitative method, a measured value that is less than the cut-off value may be rated as -, and + may be judged as positive.

By conventional patient selection methods, a patient to be administered a therapeutic agent for sepsis having an action mechanism that inhibits occurrence of TLR4-derived intracellular signaling is selected based on the patient's physiological conditions (e.g. body temperature, heart rate, respiration, leukocyte count, blood pressure, and organ failure). However, based on the action mechanism of the therapeutic agent for sepsis, the agency produces the greatest efficacy when administered at a stage where infection by living microbes occurs inside a living body, triggering LPS-induced TLR4-derived intracellular signaling. At a stage where physiological conditions such as body temperature, heart rate, respiration, leukocyte count, blood pressure, and organ failure are deteriorating, various inflammatory cytokines have already been produced in excessive amounts in response to the TLR4-derived intracellular signaling, and cytokine storms have caused systemic dysfunction, so that administration of the therapeutic agent for sepsis having an action mechanism that inhibits occurrence of TLR4-derived intracellular signaling is belated.

According to the method for selecting a patient of the invention, the efficacy of the therapeutic agent for sepsis having an action mechanism that inhibits occurrence of TLR4-derived intracellular signaling may be enhanced by selecting a patient to be administered the therapeutic agent for sepsis by using the sCD14-ST concentration in the patient's blood as index. While improvement in the survival rate is a typical index of the efficacy, the efficacy is an improvement in a patient risk, i.e., an improvement represented by at least one of decrease in the ratio of transition into a state requiring intensive care, decrease in the ratio of transition into a state requiring artificial respiration, decrease in the ratio of transition into a state requiring artificial dialysis, decrease in the ratio of transition into severe sepsis, decrease in the ratio of transition into multiple organ dysfunction, decrease in the incidence of septic shock, and the like. Improvement in physiological conditions such as body temperature, heart rate, respiration, leukocyte count, blood pressure, and organ failure may also be used as indexes of the efficacy.

The method for selecting a patient according to the invention may also be described as a method for determining a timing for administering a therapeutic agent for sepsis having an action mechanism that inhibits occurrence of TLR4-derived intracellular signaling to a patient using the sCD14-ST concentration in the patient's blood as index. Upon verification that the sCD14-ST concentration in the blood of a subject is equal to or greater than a cut-off value, the subject becomes a patient to be administered the therapeutic agent for sepsis. The method for selecting a patient and the method for determining the administration timing are thus synonymous. Thus, the present invention provides a method for determining a timing for administering a therapeutic agent for sepsis having an action mechanism that inhibits occurrence of Toll-like receptor 4-derived intracellular signaling, the method comprising a step of measuring sCD14-ST in blood derived from a subject to obtain a measured value, a step of comparing the measured value with a cut-off value, and a step of selecting the patient to be administered the therapeutic agent for sepsis when the measured value tests positive in comparison with the cut-off value. To the method for determining the administration timing according to the invention, the embodiment in the method for selecting a patient may be applied as it is.

The present invention provides a method for setting a dosage of a therapeutic agent for sepsis having an action mechanism that inhibits occurrence of intracellular signaling derived from Toll-like receptor 4. The method for setting a dosage comprises 1) a step of measuring sCD14-ST in the blood derived from a subject to obtain a measured value, 2) a step of comparing the measured value with a cut-off value, 3) a step of selecting a patient to be administered the therapeutic agent for sepsis when the measured value of the specimen tests positive in comparison with the cut-off value, and 4) a step of setting a dosage of the therapeutic agent for sepsis based on the sCD14-ST concentration in the specimen.

In the steps 1), 2), and 3) in the method for setting a dosage according to the invention, the method described in the method for selecting a patient according to the invention may be used.

In the step 4), the dosage of the therapeutic agent for sepsis is set based on the sCD14-ST concentration. In the step 3), because the patient is already selected as a patient to be administered the therapeutic agent for sepsis, administration of the therapeutic agent for sepsis to the patient may be started with at least a minimum dosage. Preferably, administration of the therapeutic agent for sepsis to the subject selected as a patient to be administered the therapeutic agent for sepsis is started with a standard dosage. When the sCD14-ST concentration has reached so high a value as, for example, at least twice the cut-off value, the therapeutic agent for sepsis is preferably administered in a high dosage ranging from a standard dosage to a maximum dosage. When the sCD14-ST concentration has reached at least five times the cut-off value, the therapeutic agent for sepsis is preferably administered in a maximum dosage.

The method for setting a dosage according to the invention may comprise a step of adjusting the dosage of the therapeutic agent for sepsis according to the progress/improvement in the pathology by monitoring the sCD14-ST concentration in the patient's blood. Where, for example, the sCD14-ST concentration has reached at least 10 times the cut-off value and, after administration of the therapeutic agent for sepsis is started with a maximum dosage, decreased to less than five times the cut-off value, the dosage of the therapeutic agent for sepsis may be changed to a standard dosage. Further, the timing for administering the therapeutic agent for sepsis, the dosage of the therapeutic agent, or the termination of the administration thereof may be selected using the index given by the sCD14-ST concentration changing to twice, five times, or ten times the preceding measured value.

The method for selecting a patient and the method for setting a dosage according to the invention may further comprise at least one of a step of measuring physiological conditions of a patient, a step of giving scores to the measured physiological conditions, a step of selecting a patient to be administered the therapeutic agent for sepsis using a combination of the patient's physiological conditions or the scores with judgment as to whether the sCD14-ST concentration in the specimen is equal to or greater than the cut-off value, a step of setting a dosage of the therapeutic agent for sepsis using a combination of the patient's physiological conditions or the scores with the sCD14-ST concentration in the specimen, a step of measuring another marker than sCD14-ST in the patient's blood, a step of judging whether the measured marker value is higher than a standard value of a normal person by comparing the measured marker value with the standard value of the normal person, a step of selecting a patient to be administered the therapeutic agent for sepsis using a combination of the measured marker value with judgment as to whether the sCD14-ST concentration in the specimen is equal to or greater than the cut-off value, and a step of setting a dosage of the therapeutic agent for sepsis using a combination of the measured marker value with the sCD14-ST concentration in the specimen. Adding physiological conditions and another marker to the judgment criteria in addition to using the sCD14-ST concentration in the blood of a patient as index enables selection of a patient having a high degree of requirement and/or urgency for administration of the therapeutic agent for sepsis and setting of a dosage of the therapeutic agent for sepsis that better suits the pathology.

The step of measuring the physiological conditions of a patient may be implemented using a known method. In a representative method, use may be made of a definition of SIRS (systemic inflammatory response syndrome) that uses body temperature, pulse rate, respiration rate or arterial carbon dioxide pressure, and leukocyte count as indexes. When at least two items out of [1] to [4] below are met, diagnosis of SIRS is given. [1] Body temperature > 38°C or < 36°C; [2] Pulse rate > 90/min; [3] Respiration rate > 20/min or PaCO₂ < 32 torr; [4] Leukocyte count > 12000/mm³ or < 4000/mm³ or immature leukocytes > 10%.

The physiological conditions of a patient may be scored using a known method. Representative examples thereof include APACHE II Score (Acute physiology and chronic health evaluation II score; Knaus WA et al., Crit Care Med, Vol.13, pp818-829 (1985)) and SOFA Score (Sepsis-related organ failure assessment score; Vincent JL et al., Intensive Care Med, Vol.22, pp707-710 (1996)).

While other markers than sCD14-ST that may be used in the step of measuring another marker than sCD14-ST in the blood of a patient may be selected as appropriate from, for example, about 170 kinds of markers described in Non-Patent Document 7, preferred examples thereof including procalcitonin, C-reactive protein (CRP), D-dimer, IL-6, TNF-α, IL-1β, IL-8, IL-1ra, and IL-10.

Examples of combinations, in which the patient's physiological conditions or the physiological scores are combined with judgment as to whether the sCD14-ST concentration in the specimen is equal to or greater than the cut-off value, used in the step of selecting a patient to be administered the therapeutic agent for sepsis, include a combination in which the sCD14-ST concentration is equal to or greater than the cut-off value, and SIRS diagnosis has been given; a combination in which the sCD14-ST concentration is equal to or greater than the cut-off value, and the APACHE II score is at least 10, preferably 15, and more preferably at least 21; and a combination in which the sCD14-ST concentration is equal to or greater than the cut-off value, and the SOFA score is at least 5, preferably at least 10.

Examples of combinations, in which the value of another marker than sCD14-ST is combined with judgment as to whether the sCD14-ST concentration in a specimen is equal to or greater than the cut-off value, used in the step of selecting a patient to be administered the therapeutic agent for sepsis include a combination in which the sCD14-ST concentration is equal to or greater than the cut-off value, and the procalcitonin concentration is at least 0.5 ng/mL, preferably at least 2.0 ng/mL; and a combination in which the sCD14-ST concentration is equal to or greater than the cut-off value, and the CRP concentration is at least 3 mg/dL, preferably at least 10 mg/dL.
In addition to the sCD14-ST concentration, a combination of the physiological conditions/physiological scores with the value of another marker than sCD14-ST may be used.

In the step of setting a dosage of the therapeutic agent for sepsis using a combination in which physiological conditions or physiological scores of a patient are combined with the sCD14-ST concentration in a specimen, the therapeutic agent for sepsis may be administered in a dosage ranging from at least a standard dosage to at most a maximum dosage when the patient's physiological conditions or physiological scores indicate a high degree of severity even though the sCD14-ST concentration is as low as about twice the cut-off value. The same applies to a combination with the value of another marker than sCD14-ST. The step of adjusting the dosage of the therapeutic agent for sepsis according to the progress/improvement in the pathology by monitoring the sCD14-ST concentration in the blood of a patient may include a combination with the physiological conditions or physiological scores and/or the value of another maker than sCD14-ST. For example, where the sCD14-ST concentration is ten times the cut-off value when administration of the therapeutic agent for sepsis is started with a maximum dosage and has in time decreased to less than five times the cut-off value, the dosage of the therapeutic agent for sepsis is set to a high dosage ranging from at least a standard dosage to at most a maximum dosage when the patient's physiological conditions or physiological scores indicate a high degree of severity as when, for example, the APACHE II score is still 21 or higher. The same applies to a combination with the value of another maker than sCD14-ST. Using a combination with the physiological conditions or physiological scores to determine the dosage when monitoring the sCD14-ST concentration in the blood of a patient is especially useful in facilitating judgment as to whether the therapeutic agent is improving systemic symptoms.

The present invention provides a sepsis treating method. The sepsis treating method of the invention is characterized in that a patient is selected using the sCD14-ST concentration in the patient's blood as index, and is a method for treating sepsis by administering a therapeutic agent for sepsis having an action mechanism that inhibits occurrence of TLR4-derived intracellular signaling to the selected patient. Specifically, sepsis is treated by administering the therapeutic agent for sepsis having an action mechanism that inhibits occurrence of TLR4-derived intracellular signaling to a patient selected by the above-described method of selecting a patient to be administered the therapeutic agent for sepsis. Sepsis may also be treated by administering the therapeutic agent for sepsis at a timing determined by the above-described method of determining a timing for administering the therapeutic agent for sepsis. Sepsis may also be treated by administering the therapeutic agent for sepsis in a dosage that is set by the above-described method for setting a dosage of the therapeutic agent for sepsis. The sepsis treating method of the invention can enhance the efficacy of a therapeutic agent for sepsis and the therapeutic efficiency.

The present invention provides a therapeutic agent for sepsis having an action mechanism that inhibits occurrence of TLR4-derived intracellular signaling and characterized in that the therapeutic agent is used by being administered to a subject suspected to have sepsis when a measured value obtained by measuring sCD14-ST in a blood specimen derived from the subject and compared with a cut-off value tests positive in comparison with the cut-off value. The therapeutic agent for sepsis of the invention is characterized in that the therapeutic agent is used by being administered to a patient selected by the above-described method for selecting a patient. The therapeutic agent for sepsis of the invention may also be used by being administered at a timing determined by the above-described method for determining the administration timing. The therapeutic agent for sepsis of the invention may also be used by being administered in a dosage set by the above-described method for setting a dosage. Specifically, the therapeutic agent for sepsis having an action mechanism that inhibits occurrence of TLR4-derived intracellular signaling is at least one selected from the group consisting of Resatorvid, E5564, and F1024S-D2(3). The therapeutic agent for sepsis can produce enhanced efficacy and therapeutic effects when used by being administered by the method and in the dosage specifically described above.

The present invention is more specifically described below with reference to examples, which are given by way of illustration only and should not be construed as limiting the invention.

### Examples

### (Example 1) Selection of Subject to be administered Therapeutic Agent for Sepsis (F1024S-D2(3)) and Setting of Dosage Thereof using sCD14-ST Concentration as Index

A cecal ligation and puncture-induced peritonitis rabbit model (rabbit CLP model; CLP for cecal ligation and puncture) was used as the sepsis model to verify the improvement in efficacy of the therapeutic agent for sepsis when a patient to be administered the therapeutic agent for sepsis (F1024S-D2(3)) was selected using the sCD14-ST concentration as index. The rabbit CLP model was prepared using male rabbits, New Zealand white, each weighing 1.5 kg to 1.99 kg on arrival (Kitayama Labes Co., Ltd.), which had their cecum punctured under anesthesia at two places to make holes each measuring 2 cm to release the cecal contents into the peritoneal cavity according to the method described by Keith A et al (Journal of Surgical Research, Vol. 29, 189 (1980)). In order to stabilize the postsurgical state, all the operated individuals were intravenously administered 60 mg/kg of Shiomarin (Latamoxef Sodium for administration by injection, Shionogi & Co., Ltd.) two hours after surgery.
Blood was sampled at intervals before and after surgery to measure the sCD14-ST concentrations in the blood. The sCD14-ST concentrations in the blood were measured by the method described in Example 3 in WO2009/142303. In this model, the pathology progresses within several hours, resulting in death within 7 to 8 hours after surgery in the earliest cases. Because the measured values of the sCD14-ST concentration in the blood can only be known five to six hours after blood sampling, administering the therapeutic agent only after checking the results is belated. Therefore, the administration timing of the therapeutic agent was set to two hours after surgery, and the blood sCD14-ST concentration at the time when the therapeutic agent was administered was later checked with reference to blood sCD14-ST concentration transition data. Using the respective sCD14-ST concentrations in the blood before surgery of the individuals as normal values, twice the normal values were set as the respective cut-off values. Thus, the survival rates of the cases where the sCD14-ST concentrations in the blood were at least twice the normal values at the time of administration of the therapeutic agent were compared with that of the control cases (see Table 1). The therapeutic agent-administered cases were intravenously administered 10 mg/kg of F1024S-D2(3). The control cases were intravenously administered 10 mg/kg of human immunoglobulin (hIg) in lieu of F1024S-D2(3).

**[Table 1]**

| Case No. | Severity | Administered drug | Blood sCD14-ST concentration (pg/ml) | | | | Survival time |
|---|---|---|---|---|---|---|---|
| | | | Pre-surgical | On administration | 3 hr after administration | 6 hr after administration | |
| 1 | Medium | F1024S-D2 (3) | 96 | 370 | 497 | 91 | 47 hr |
| 2 | Medium | hIg (control) | 94 | 498 | 603 | 316 | 21 hr |
| 3 | High | F1024S-D2(3) | 56 | 124 | 1156 | 952 | 23 hr |
| 4 | High | hIg (control) | 140 | 154 | 1318 | 685 | 9 hr |
| 5 | especially high | F1024S-D2(3) | 44 | 2925 | 521 | - | 7 hr |
| 6 | Especially high | hIg (control) | 63 | 1791 | 396 | - | 7 hr |

The transition of the sCD14-ST concentration in the blood reflects the severity of sepsis. In this test, three major types of concentration transition were observed as follows: A) a medium severity type wherein the sCD14-ST concentration increased to a medium degree immediately after surgery and, after remaining at a level for a while, gradually decreases; B) a high severity type wherein the sCD14-ST concentration showed a gradual increase immediately after surgery, followed by a significantly high sCD14-ST concentration; and C) a specially high severity type wherein the surgery was immediately followed by a significantly high sCD14-ST concentration. Comparison between the therapeutic agent-administered cases and the control cases was made between cases exhibiting similar severity levels indicated by the transition of the sCD14-ST concentration in the blood.
As a result, as shown in Table 1, an effect of prolonging the survival time was observed in the medium to high severity cases when F1024S-D2(3) was administered at the time the blood sCD14-ST concentration reached at least twice the normal value. On the other hand, in the especially high severity cases, no difference in survival time was observed between the therapeutic agent-administered cases and the control cases. This may be attributed to the fact that the severity in such cases had already so advanced at the time of administration of the therapeutic agent, with the sCD14-ST concentrations in the blood at significantly high levels (at least 20 times the presurgical normal values), that the therapeutic agent was incapable of producing therapeutic effects. For the therapeutic agent to produce effects in such cases, increase in dosage, for example, would be required. The results related to the high severity cases implies that administration of the therapeutic agent effected, when possible, at a timing before the sCD14-ST concentration in the blood has increased to a significantly high level can improve the survival rate. It may be said that even in especially high severity cases, early detection of increase in the sCD14-ST concentration in the blood and administration of the therapeutic agent effected at an earlier timing may improve the survival rate.
The above results suggest that selecting a patient to be administered the therapeutic agent for sepsis, determining an administration timing, and setting a dosage using the sCD14-ST concentration in the blood as index are useful for enhancing the efficacy of the therapeutic agent for sepsis.

### (Example 2) Selection of Subject to be Administered Therapeutic Agent for Sepsis (Resatorvid), Determination of Administration Timing, and Setting of Dosage Thereof Using sCD14-ST Concentration as Index

### 2-1. Rabbit Sepsis Model

A rabbit CLP model is prepared as in Example 1. The Resatorvid administration timing is set to 1 hour and 5 hours after surgery or 2 hours and 6 hours after surgery and, as in Example 1, the blood sCD14-ST concentration at the time the therapeutic agent is administered is later checked with reference to the blood sCD14-ST concentration transition data. The dosage of Resatorvid is 3 mg/kg to 10 mg/kg. The body temperature and the leukocyte count of the rabbits are measured with time and used as indexes of the physiological conditions.
Comparison of the therapeutic agent-administered cases and the control cases reveals improvement in the efficacy produced when the sCD14-ST concentration is used as index in selection of a patient to be administered Resatorvid, determination of an administration timing, and setting of a dosage.

### 2-2. Human Clinical Test

Resatorvid was administered to patients with severe sepsis and a symptom of shock or respiratory failure, with the result that Resatorvid failed to suppress the serum IL-6 level, and that while a tendency to reduce the mortality rate was observed in the high-dosage group, it was of no statistic significance (Todd W. Rice et al., Crit Care Med, 38, pp1685-1694 (2010)). The specific standards used for selection of patients are as follows: Severe sepsis meets at least three items of the SIRS diagnostic criteria [1] to [4], and the patient has or is suspected to have an infection requiring treatment with antibiotics. Septic shock needs a vasopressor to maintain a systolic blood pressure of at least 90 mmHg even under an appropriate body fluid management. Respiration failure exhibits a PaO₂/FIO₂ ratio of less than 200 even with artificial respiration.
On the other hand, when the patient selection standards are set using the sCD14-ST concentration as index, improvement in Resatorvid efficacy can be observed. According to one example of the patient selection standards, Resatorvid is administered to a patient who meets at least three items of the SIRS diagnostic criteria [1] to [4] and whose sCD14-ST concentration is equal to or greater than the cut-off value. Addition of the sCD14-ST concentration to the indexes used in setting the patient selection standards enables establishment of the patient selection standards that suit the action mechanism of Resatorvid and thus enables further improvement in Resatorvid efficacy than when the patient selection standards are set using only the physiological conditions as indexes.

### (Example 3) Selection of Subject to be Administered Therapeutic Agent for Sepsis (E5564), Determination of Administration Timing, and Setting of Dosage Thereof Using sCD14-ST Concentration as Index

### 3-1. Rabbit Sepsis Model

A rabbit CLP model is prepared as in Example 1. The E5564 administration timing is set to 1 hour and 5 hours after surgery or 2 hours and 6 hours after surgery and, as in Example 1, the sCD14-ST concentration in the blood at the time the therapeutic agent is administered is later checked with reference to the blood sCD14-ST concentration transition data. The dosage of E5564 is 3 mg/kg to 10 mg/kg. The body temperature and the leukocyte count of the rabbits are measured with time and used as indexes of the physiological conditions.
Comparison of the therapeutic agent-administered cases and the control cases reveals improvement in the efficacy produced when the sCD14-ST concentration is used as index in selection of a patient to be administered E5564, determination of an administration timing, and setting of a dosage.

### 3-2. Human Clinical Test

E5564 was administered to severe septic patients whose mortality risk was estimated at 20 % to 80 % by APACHE II Score, with the result that a tendency to reduce the mortality rate was observed in a high-dosage group, but it was of no statistic significance (Mark Tidswell et al., Crit Care Med, 38, pp72-83 (2010). The mortality risk estimated by APACHE II Score was calculated according to Knaus WA et al., Crit Care Med, Vol.13, pp818-829 (1985). Severe sepsis meets at least three items of the SIRS diagnostic criteria [1] to [4] and is accompanied by at least one kind of septic organ failure selected from the group consisting of shock, kidney failure, liver failure and metabolic acidosis.
On the other hand, when the patient selection standards are set using the sCD14-ST concentration as index, improvement in E5564 efficacy can be observed. According to one example of the patient selection standards, E5564 is administered to a patient who meets at least three items of the SIRS diagnostic criteria [1] to [4] and whose sCD14-ST concentration is equal to or greater than the cut-off value. Addition of the sCD14-ST concentration to the indexes used in setting the patient selection standards enables establishment of the patient selection standards that suit the action mechanism of E5564 and thus permits further improvement in E5564 efficacy than when the patient selection standards are set using only the physiological conditions as indexes.

### (Example 4) Selection of Subject to be Administered Therapeutic Agent for Sepsis (Resatorvid) and Determination of Administration Timing Using sCD14-ST Concentration as Index

When the sCD14-ST concentration was used as index in selection of a patient to be administered the therapeutic agent for sepsis (Resatorvid) and determination of an administration timing using a rabbit CLP model as the sepsis model, improvement in efficacy was observed.

### 4-1. Establishment of Resatorvid-administered Rabbit CLP Model

A rabbit CLP model was prepared as in Example 1, and all the operated individuals were intravenously administered 60 mg/kg of Shiomarin one hour after surgery in order to stabilize the postsurgical state (changing the Shiomarin administration timing from two hours after surgery to one hour after surgery did not affect the survival rate).
Two Resatorvid-administered groups were set with different administration timings. Ten mg/kg of Resatorvid was intravenously administered at timings of 1 hour and 5 hours after surgery to one group (hereinafter referred to as first-hour administered group) and 3 hours and 7 hours after surgery to the other group (hereinafter referred to as third-hour administered group). A group not to be administered Resatorvid (hereinafter referred to as control group) was administered a 10% aqueous solution of cyclodextrin, a Resatorvid solvent, 1 hour and 5 hours after surgery. Each of the groups consisted of 10 individuals; survival was checked until 48 hours after surgery. The platelet count and the leukocyte count were measured until 24 hours after surgery.

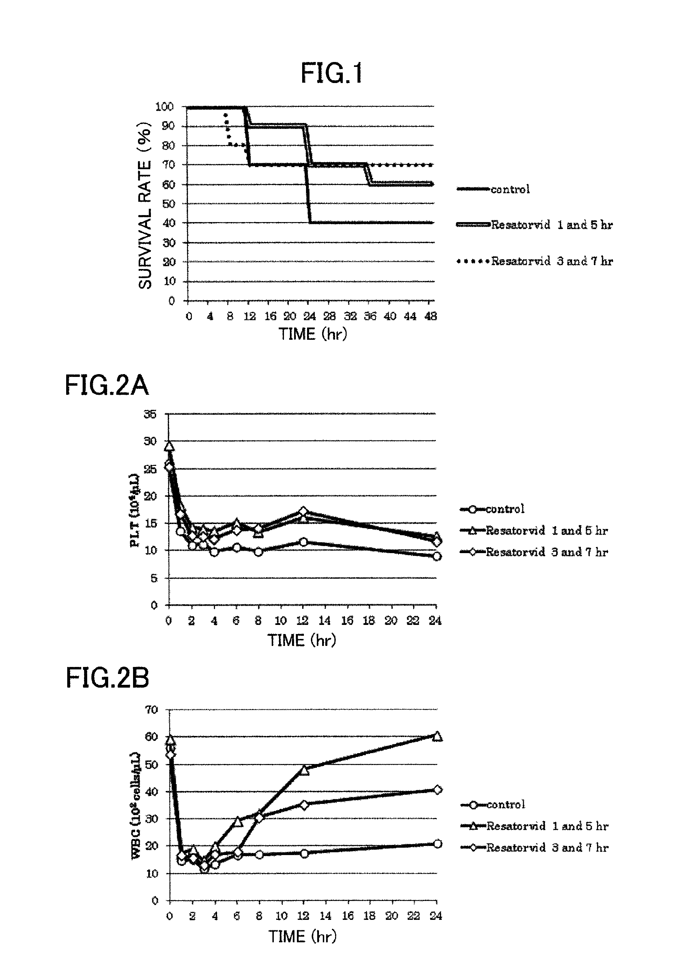

FIG. 1 shows the respective survival rates of the groups. The survival rates improved in the first-hour Resatorvid-administered group and the third-hour Resatorvid-administered group over the control group. In the first 24 hours, the first-hour administered group showed a higher survival rate than the third-hour administered group.
The platelet count transitions in the respective groups are shown in FIG. 2A; the leukocyte count transitions in the respective groups are shown in FIG. 2B. Decreases in platelet count and leukocyte count are pathological conditions observed in severe septic patients. At the 24th hour after surgery, the Resatorvid-administered groups showed recovery in leukocyte count as compared with the control group. The effects of enabling recovery in leukocyte count were observed more significantly in the first-hour administered group than in the third-hour administered group. As regards the platelet count, the Resatorvid-administered groups showed a tendency to recover at the 12th hour after surgery, but failed to recover.
From the above results, establishment of a system that allows verification of the Resatorvid efficacy using the rabbit CLP model was achieved. Further, the above results suggest that Resatorvid administered at an earlier timing produces greater efficacy.

### 4-2. Determination of Therapeutic Agent Administration Timing Using sCD14-ST Concentration as Index

In the Resatorvid-administered rabbit CLP model, comparison as to how many hours after surgery the selection of a patient to be administered the therapeutic agent becomes possible was made between a case where the sCD14-ST concentration was used as index and a case where the body temperature, a physiological condition, was used as index. This simulates selection of a patient to be administered the therapeutic agent using physiological conditions as indexes for judgment as practiced in clinical settings where human septic patients are treated. In the case of humans, for example, when at least two items out of (1) to (4) below are met, diagnosis of SIRS is given. [1] Body temperature > 38°C or < 36°C; [2] Pulse rate > 90/min; [3] Respiration rate > 20/min or PaCO₂ < 32 torr; [4] Leukocyte count > 12000/mm³ or < 4000/mm³ or immature leukocytes > 10%. Because decrease in leukocyte count is observed immediately after surgery in the rabbit CLP model, elevation in body temperature was used as index of physiological conditions.

The averages of the sCD14-ST concentration and the body temperature before surgery were 112 pg/mL and 40.2°C respectively in the control group. These values are used as the normal values. The cut-off value of the sCD14-ST concentration was set to twice the normal value or 224 pg/mL. The cut-off value of the body temperature elevation was set to 41.5 °C so as to detect an elevation in body temperature by at least 1 °C from the normal value. A group in which the sCD14-ST concentration was measured as time elapsed after surgery is referred to as sCD14-ST concentration-measured patient group (indicated as "sCD14-ST" in the figure), and a group in which the body temperature was measured as time elapsed after surgery is referred to as body temperature-measured patient group (indicated as "Body temp." in the figure).

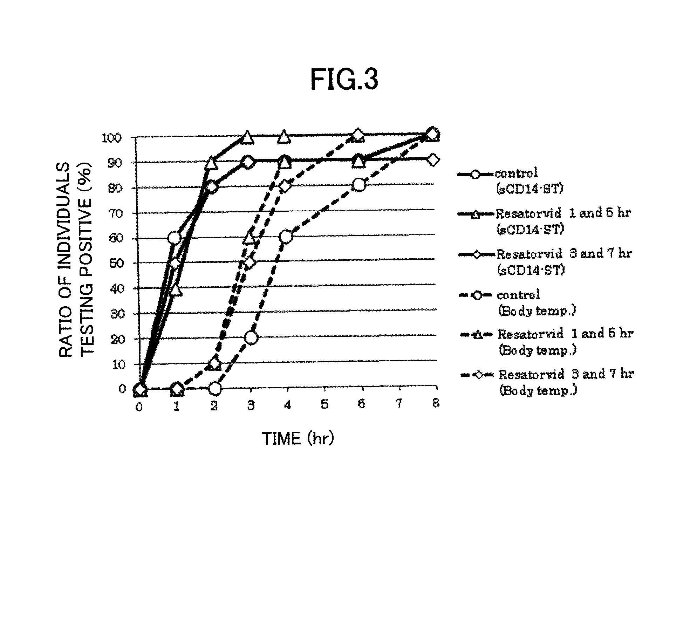

FIG. 3 shows temporal changes in the number of individuals that tested positive with respect to the cut-off values of the sCD14-ST concentration and the body temperature elevation. The horizontal axis of FIG. 3 indicates time that elapsed after surgery; the vertical axis indicates the ratio of the individuals that tested positive. In sCD14-ST concentration, about 50 % of the individuals tested positive at the first hour after surgery, and about 80 % of the individuals tested positive at the second hour after surgery. In body temperature elevation, about 40% of the individuals tested positive at the third hour after surgery, and about 70% of the individuals tested positive at the fourth hour after surgery
It may be said that when the sCD14-ST concentration is used as index in determining the therapeutic agent administration timing, administration may be started to most of the individuals at the first to the second hour after surgery, and that excellent effects of improving the survival time and enabling recovery in leukocyte count as observed in the first-hour Resatorvid-administered group can be obtained.

## Claims

1. A method for selecting a patient to be administered a therapeutic agent for sepsis having an action mechanism that inhibits occurrence of Toll-like receptor 4-derived intracellular signaling, the method comprising the steps of:
measuring sCD14-ST in a blood specimen derived from the subject,
comparing the measured value with a cut-off value, and
selecting the patient to be administered the therapeutic agent for sepsis when the measured value of the specimen is equal to or higher than the cut-off value and thus tests positive in comparison with the cut-off value.

2. A method for determining a timing for administering a therapeutic agent for sepsis having an action mechanism that inhibits occurrence of Toll-like receptor 4-derived intracellular signaling, the method comprising the steps of:
measuring sCD14-ST in a blood specimen derived from a subject,
comparing the measured value with a cut-off value, and
selecting the subject as a patient being in a timing to be administered the therapeutic agent for sepsis when the measured value of the specimen tests positive in comparison with the cut-off value.

3. A method for setting a dosage of a therapeutic agent for sepsis having an action mechanism that inhibits occurrence of Toll-like receptor 4-derived intracellular signaling, the method comprising the steps of:
measuring sCD14-ST in a blood specimen derived from a subject,
comparing the measured value with a cut-off value, a step of selecting the subject as a patient to be administered the therapeutic agent for sepsis when the measured value of the specimen tests positive in comparison with the cut-off value, and
setting a dosage of the therapeutic agent for sepsis based on the sCD14-ST concentration in the specimen.

4. The method according to any one of Claims 1 to 3, wherein the therapeutic agent for sepsis having the action mechanism that inhibits occurrence of Toll-like receptor 4-derived intracellular signaling is at least one selected from the group consisting of Resatorvid, E5564, and F1024S-D2(3).

5. A therapeutic agent for sepsis having an action mechanism that inhibits occurrence of Toll-like receptor 4-derived intracellular signaling, the therapeutic agent being administered to a subject suspected to have sepsis when a measured value obtained by measuring sCD14-ST in a blood specimen derived from the subject and compared with a cut-off value tests positive in comparison with the cut-off value.

6. A therapeutic agent for sepsis having an action mechanism that inhibits occurrence of Toll-like receptor 4-derived intracellular signaling, a dosage being set according to a concentration of sCD14-ST in a blood specimen derived from a subject suspected to have sepsis when a measured value of the specimen obtained by measuring sCD14-ST in the blood specimen and compared with a cut-off value tests positive in comparison with the cut-off value.

7. The therapeutic agent for sepsis having the action mechanism that inhibits occurrence of Toll-like receptor 4-derived intracellular signaling according to Claim 5 or 6, wherein the therapeutic agent for sepsis having the action mechanism that inhibits occurrence of Toll-like receptor 4-derived intracellular signaling is at least one selected from the group consisting of Resatorvid, E5564, and F1024S-D2(3).


### Sequence Listing

SEQUENCE LISTING

<110>  MOCHIDA PHARMACEUTICAL CO., LTD.

<120>  A METHOD FOR SELECTING A PATIENT TO ADMINISTER A THERAPEUTICUM OF
SEPSIS

<130>  W-4638PCT

<160>  3

<170>  PatentIn version 3.1

<210>  1
<211>  11
<212>  PRT
<213>  Artificial sequence

<220>
<223>  N-terminal sequence of Human CD14

<400>  1

Thr Thr Pro Glu Pro Cys Glu Leu Asp Asp Glu
1               5                   10


<210>  2
<211>  16
<212>  PRT
<213>  Artificial sequence

<220>
<223>  Sites 53-68 of Human CD14

<400>  2

Arg Val Asp Ala Asp Ala Asp Pro Arg Gln Tyr Ala Asp Thr Val Lys
1               5                   10                  15


<210>  3
<211>  356
<212>  PRT
<213>  Homo sapiens

<400>  3

Thr Thr Pro Glu Pro Cys Glu Leu Asp Asp Glu Asp Phe Arg Cys Val
1               5                   10                  15


Cys Asn Phe Ser Glu Pro Gln Pro Asp Trp Ser Glu Ala Phe Gln Cys
            20                  25                  30


Val Ser Ala Val Glu Val Glu Ile His Ala Gly Gly Leu Asn Leu Glu
        35                  40                  45


Pro Phe Leu Lys Arg Val Asp Ala Asp Ala Asp Pro Arg Gln Tyr Ala
    50                  55                  60

Asp Thr Val Lys Ala Leu Arg Val Arg Arg Leu Thr Val Gly Ala Ala
65                  70                  75                  80

Gln Val Pro Ala Gln Leu Leu Val Gly Ala Leu Arg Val Leu Ala Tyr
                85                  90                  95

Ser Arg Leu Lys Glu Leu Thr Leu Glu Asp Leu Lys Ile Thr Gly Thr
            100                 105                 110

Met Pro Pro Leu Pro Leu Glu Ala Thr Gly Leu Ala Leu Ser Ser Leu
        115                 120                 125

Arg Leu Arg Asn Val Ser Trp Ala Thr Gly Arg Ser Trp Leu Ala Glu
    130                 135                 140

Leu Gln Gln Trp Leu Lys Pro Gly Leu Lys Val Leu Ser Ile Ala Gln
145                 150                 155                 160

Ala His Ser Pro Ala Phe Ser Cys Glu Gln Val Arg Ala Phe Pro Ala
                165                 170                 175

Leu Thr Ser Leu Asp Leu Ser Asp Asn Pro Gly Leu Gly Glu Arg Gly
            180                 185                 190

Leu Met Ala Ala Leu Cys Pro His Lys Phe Pro Ala Ile Gln Asn Leu
        195                 200                 205

Ala Leu Arg Asn Thr Gly Ile Glu Thr Pro Thr Gly Val Cys Ala Ala
    210                 215                 220

Leu Ala Ala Ala Gly Val Gln Pro His Ser Leu Asp Leu Ser His Asn
225                 230                 235                 240

Ser Leu Arg Ala Thr Val Asn Pro Ser Ala Pro Arg Cys Met Trp Ser
                245                 250                 255

Ser Ala Leu Asn Ser Leu Asn Leu Ser Phe Ala Gly Leu Glu Gln Val
            260                 265                 270

Pro Lys Gly Leu Pro Ala Lys Leu Arg Val Leu Asp Leu Ser Cys Asn
        275                 280                 285

Arg Leu Asn Arg Ala Pro Gln Pro Asp Glu Leu Pro Glu Val Asp Asn
    290                 295                 300

Leu Thr Leu Asp Gly Asn Pro Phe Leu Val Pro Gly Thr Ala Leu Pro
305                 310                 315                 320

His Glu Gly Ser Met Asn Ser Gly Val Val Pro Ala Cys Ala Arg Ser
                325                 330                 335

Thr Leu Ser Val Gly Val Ser Gly Thr Leu Val Leu Leu Gln Gly Ala
            340                 345                 350

Arg Gly Phe Ala
        355